# EUROPÄISCHE PATENTSCHRIFT

(11) **EP 0 758 548 B1**
(45) Veröffentlichungstag und Bekanntmachung des Hinweises auf die Patenterteilung: **04.10.2000**
(21) Anmeldenummer: 96111436.0
(22) Anmeldetag: 16.07.1996
(51) Int. Cl.: A61K 31/165, A61K 31/245, A61K 31/445, A61K 9/70

(54) **Lokalanästhetika zur topischen Therapie von Rückenschmerzen und Muskelverspannung**
Local anesthetics for topical treatment of back pain and muscle tension
L'application topique d'anesthésiques locaux pour traiter des douleurs dorsales et de la raideur musculaire

(30) Priorität: 17.07.1995 DE 19526031
(43) Veröffentlichungstag der Anmeldung: 19.02.1997
(73) Patentinhaber: EpiCept Corporation, Englewood Cliffs, N.J. 07632 (US)
(72) Erfinder: Liedtke, Rainer K., Dr., 82027 Grünwald (DE)
(74) Vertreter: Tiedtke, Harro, Dipl.-Ing.

(56) Entgegenhaltungen:
- WO-A-92/15289
- WO-A-93/17674
- ACTA-DERMATO-VENEREOLOGICA, Bd. 70, Nr. 4, 1990, Seiten 314-318, XP000603746 L. ARENDT-NILESEN: "Regional variations in analgesic efficacy of EMLA cream"

## Beschreibung

Die Erfindung betrifft die Verwendung eines Lokal anästhetikums zur Herstellung einer träger-gebundenen Zusammensetzung für eine topischen Therapie von Rückenschmerzen, Muskelverspannung und myofascialem Schmerz.

Es ist bekannt, daß Rückenschmerzen, Muskelverspannungen und myofascialer Schmerz überwiegend auf neurologischen und muskulären Ursachen beruht, wobei u.a. psychosomatische Stressfaktoren, physikalische Umweltfaktoren, unfunktionelle aktive oder passive Fixierung in der Körperhaltung, mangelnde Bewegung, belastungsabhängige funktionelle oder organische Störungen der Wirbelsäulenfunktion, z.B. Bandscheibenschädigungen, involviert sind.

Die Indidenz von Rückenschmerzen beträgt ca. 80% der Bevölkerung, d.h. dieser Anteil hat, in unterschiedlicher Häufigkeit, Rückenschmerzen. Rückenschmerzen gehören auch zu den ökonomisch wichtigsten Ursachen bezüglich des Ausfalls von Arbeitszeiten.
Die Symptomatiken werden pharmakologisch überwiegend mit systemisch wirksamen, nichtopioiden, oralen oder injektablen, Analgetika und Antiphlogistika behandelt, partiell in Kombination mit psyschosomatischen oder physikalischen Behandlungen, teils auch mit anderen Methoden z.B. Akupunktur. Letzte Konsequenz bei Bandscheibenerkrankungen sind operative Maßnahmen.
Diese Pharmakotherapien stellen aber insgesamt noch keine ausreichend verträgliche und effektive Behandlungsformen dar. Als pharmakologisches Prinzip werden insbesondere Derivate der Salicysäure, vorzugsweise Acetylsaliclsäure, nichtsteroidale Antiphlogisika, z.B. Ibuprofen, oder Anilin-Derivate, z.B. Paracetamol, eingesetzt (e.g. Brune K. Beck W. in: M. Zenz, I. Jura (Edits.) Lehrbuch der Schmerztherapie, WFG, Stuttgart 1993, S.121-135). Weiterhin wird der Einsatz zentraler oder peripherer Muskelrelaxantien vorgenommen, auch der von Tranquillantien der Benzodiazepin-Gruppe oder verschiedener Antidepressiva.
Allen systemischen analgetischen wie auch muskelrelaxierenden Therapien gemeinsam ist eine erhebliche Qoute unerwünschter Wirkungsquote. Bei Salicylsäure-Derivaten und nichtsteroidalen Antiphlogistika finden sich insbesondere erhebliche und häufige Magenstörungen, als Folge des antiproliferativen Wirkungsmechanismus. Bei dem hier schwächer wirksamen Paracetamol findet sich eine metabolische Belastung von Leber- und Nieren-Funktionen, insbesondere bei dem länger und erforderlich höher dosierten Gebrauch. Bei Muskelrelaxantien findet sich insbesondere eine hohe Rate an Sedierung, gastrointestinalen Störungen sowie auch Paresen. Die Anwendung dieser systemischen Therapien wird somit durch das jeweilige Spektrum der produktspezifischen unerwünschten Wirkungen limitiert, da sich systemische Interventionen alle Organe und Organsysteme einbeziehen.

Als ein besser wirksames pharmakologisches Prinzip wäre eine geeignete Form der Anwendung niedrig dosierter Lokalanästhetika zu betrachten. Amid- und Ester-Lokalanästhetika, z.B. das Lidocain vom Amid-Typ, zeigen als pharmakologischen Wirkungsmechanismus eine Hemmung des schnellen Natrium Ionen-Influx in Nervenfasern. Sie blockieren hierüber die Impulsleitung der Nervenbahn, was prinzipiell alle regionalen Nervenfasern betrifft. Die sensorischen, anatomisch dünneren, Fasern sind infolge ihrer Morphologie empfindlicher als die motorischen Fasern (Strichartz, G.R. (Ed.) Local Anesthetics, Handbook of Experimental Pharmacology, Vol. 81, Springer, Berlin - New York, 1987). Hieraus lassen sich auch Wirkungseffekte differenzieren.

Eine systemische Anwendung von Lokalanästhetika wäre invasiv, mittels Injektionen möglich. Dies entfällt aber schon weitgehend auf Grund einer Gefahr systemischer Überdosierung mit u.a. ernsten kardialen Nebenwirkungen. Direkte Anwendung von Lokalananästhetika durch lokale Injektionen ist technisch möglich und wird auch verschiedentlich ausgeführt. Doch sind lokale Injektionen nicht nur schmerzhaft, sie können auch von den Patienten in keinem Fall selbst ausgeführt werden. Die lokal oberflächliche Injektionstechnik beruht auf der sogenannten Neuraltherapie mit muskulären Triggerpunkten und setzt eine erfahrene ärzliche Handhabung und Technik voraus (J.T. Travell, D.G. Simons, Myofascial Pain and Dysfunction, Vol I/II, Williams & Wilkins, Baltimore, 1983). Sie ist daher auf den Einsatz von klinisch schwerwiegenderen Störungen begrenzt. Der Einsatz konventioneller topischer Formulierungen, z.B. Cremes, erlaubt weder eine exakte Dosierung noch kontinuierliche Penetration über eine längere Anwendungsdauer.

Die WO-A-93/17 674 beschreibt die Verwendung eines oder mehrerer Lokalanästhetika zur Herstellung einer pharmazeutischen Zusammensetzung für die topische Behandlung von rheumatoider Arthritis (RA). Konkret werden die Gelenkregionen der Hand behandelt. Zur Applikation wird die Lokalanästhetika-haltige Zusammensetzung z.B. in Form eines Gelees injiziert oder in Form einer Creme oder über einen Träger aufgetragen.

Die WO-A-92/15 289 beschreibt Zusammensetzungen zur topischen Applikation von pharmazeutisch wirksamen Mitteln über formbeständige Träger. Als pharmazeutisch wirksame Mittel werden neben Lokalanästhetika weitere Arzneimittelklassen in Betracht gezogen. Die trägergebundene Zusammensetzung ist gekennzeichnet durch die Anwesenheit eines Lösungsmittels für das Arzneimittel, eines bioverträglichen Klebstoffs sowie eines Weichmachers hierfür, so dass die Zusammensetzung im wesentlichen wasserfrei ist und das Arzneimittel nicht-kristallin vorliegt. Mit der Lokalanästhetika-haltigen Zusammensetzung soll möglichst schnell ein lokaler Betäubungszustand erreicht werden. Konkret geht es um die Lokalanästhesie im Mund- bzw. Backenbereich, wobei - vorzugsweise über die Schleimhaut - eine Zahnbetäubung beabsichtigt ist.

Die wissenschaftliche Studie aus "Acta Dermato-Venereologica", 1990, 70(4), S. 314-318 beschreibt lokale Unterschiede in der analgetischen Wirksamkeit der an sich bekannten EMLA-Creme. Dies wird an unterschiedlichen Hautregionen untersucht, nämlich Stirn, Wange, Rücken, Armbeuge und Handrücken. Dabei wird auf die für EMLA typischen Indikationen Bezug genommen, die sich durch die präventive Betäubung der Haut vor kleinen chirurgischen Eingriffen auszeichnen.

Der Erfindung liegt die Aufgabe zugrunde, die Therapie der Symptomatik von Rückenschmerzen, Muskelverspannungen und myofascialem Schmerz zu verbessern.

Diese Aufgabe wird durch die im Patentanspruch 1 wiedergegebene Verwendung gelöst. Dabei wird ein für intakte Rückenhaut oder äußere Gelenkshaut geeignetes topisches Trägersystem eingesetzt, das mit einer therapeutischen Dosis eines Lokalanästhetikums beladen ist und das Lokalanästhetikum gezielt auf die unter dem topischen Trägersystem befindliche Hautregion aufbringt.

Um Wirksamkeit und Verträglichkeit der topischen Therapie zu verbessern sind, in einer weiteren Ausbildung der Erfindung Lokalanästhetika vom Amid- oder Ester-Typ enthalten, insbesondere Lidocain, Tetracain, Bupivacain, Prilocain, Mepivacain, Etidocain sowie Procain und Benzocain, wobei diese Stoffe in Konzentrationsbereichen von 0,5%-40% vorliegen.

Um Wirksamkeit und Verträglichkeit der Therapie zu verbessern werden, in einer weiteren Ausbildung der Erfindung, in dem eingesetzten topischen Trägersytem 2 Lokalanästhetika mit unterschiedlicher Pharmakokinetik kombiniert, wobei diese Einzelstoffe in solchen Konzentrationen vorliegen, daß die Gesamkonzentration beider Wirkstoffe nicht mehr als 40% beträgt.

Um die Therapie insgesamt sicherer und besser handhabbar zu machen, liegt, in einer weiteren Ausbildung der Erfindung, das topische Trägersystem in solchen Formen vor, die den speziellen Gegebenheiten des Applikationsfeldes von Rückenhaut oder äußerer Gelenkshaut entsprechen, und die äußere Form des topischen Trägersystems rund, oval, eckig, mit konkaven oder konvexen Aussenformen ist, oder das Trägersystem auch mit oder ohne zusätzliche Hilfsmittel vom Anwender in entsprechende Formen segmentiert werden kann.

Eine Therapie von Rückenschmerz, Muskelverspannung und myofascialem Schmerz mittels eines auf sogenannte Triggerpunkte applizierten Trägersystems mit Lokalanästhetika wurde bisher noch nicht beschrieben. Als ein mit der Erfindung erzielter Vorteil ergibt sich daher, daß mit diesem neuen Prinzip erstmals auch eine nichtinvasive und lokale Behandlungsmöglichkeit von Symptomatiken des Rückenschmerzes, Muskelverspannungen und myofascialem Schmerz gegeben ist.

Die topische Therapie mit Lokalanästhetika in einem Trägersystem ermöglicht eine lokal gezielte und dauerhafte therapeutische Beeinflussung terminal und funktionell vernetzter Nervenbahnen im oberflächlichen Hautbereich.

Da dieser Bereich auch über eine gute cutane Resorptionskapazität verfügt, kann zudem mit nur geringen topischen Dosierungen vorgegangen werden. Systemische Gefährdung, wie bei üblich oralen oder injektablen Analgetika, Antiphlogistika oder Muskelrelaxantien gegeben, wird vermieden, da die Lokalanästhetika, z.B. Lidocain, bei der retardierten cutanen Aufnahme weit überwiegend metabolisiert werden, so daß auch keine systemischen Wirkspiegel mit entsprechenden Organbelastungen auftreten.

Die Therapie ist über geringe Dosen steuerbar und längeranhaltend und kann nach Bedarf durch Abnahme des Trägers unterbrochen werden.

Insgesamt weist die topische Therapie von Rückenschmerz, Muskelverspannung und myofascialem Schmerz somit nicht die unerwünschten Nebeneffekte auf wie systemische Therapien mit u.a. Analgetika, Antiphlogistika oder Muskelrelaxantien, da vorherige Wirksstoff-Distribution über den gesamten Körper vermieden wird, mit der diese die anderen Organe und Organsysteme belasten. Zudem verfügen Lokalanästhetika auch über eine gute lokale Gewebeverträglichkeit.

Als Beispiel für eine technisch geeignete Ausgestaltung eines topischen Trägersystems mit Lokalanästhetika für die Therapie der Symptomatik von Rückenschmerz, Muskelverspannung und myofascialem Schmerz wird auf die Beschreibungen der technischen Trägersysteme US-A-4,765,986, EP-A-0205974, DE-A-3716575 und DE-A-3811564 verwiesen, jedoch ohne es damit auf diese beschriebenen Techniken beschränken zu wollen.

Zur topischen Therapie von Symptomatiken von Rückenschmerzen, Muskelverspannung und myofascialem Schmerz, werden für die Rückenhaut und Gelenkshaut geeignete, topische Trägersysteme mit einer therapeutischen Dosis an Lokalanästhetika eingesetzt.

Die topische Therapie mit einem Trägersystem mit Lokalanästhetika stellt eine neue nichtinasive, nebenwirkungsarme und effektive, Behandlungsmöglichkeit für die Symptomatik von Rückenschmerzen, Muskelverspannung und myofascialem Schmerz dar.

## Patentansprüche

1. Verwendung eines Lokalanästhetikums zur Herstellung einer Zusammensetzung für die topische Behandlung von Rückenschmerzen, Muskelverspannungen und myofascialem Schmerz, wobei die Lokalanästhetikum-enthaltende Zusammensetzung in ein für die intakte Rückenhaut oder für die äußere Gelenkshaut geeignetes, topisches Trägersystem eingebracht wird, so dass das Trägersystem mit einer therapeutischen Dosis des Lokalanästhetikums beladen ist und in der Lage ist, das Lokalanästhetikum gezielt auf die unter dem topischen Trägersystem liegende Hautregion aufzubringen.

2. Verwendung nach Anspruch 1, dadurch gekennzeichnet, dass in dem topischen Trägersystem Lokalanästhetika vom Amid- oder Ester-Typ eingesetzt werden, insbesondere Lidocain, Tetracain, Prilocain, Bupivacain, Mepivacain, Etidocain sowie Procain und Benzocain, wobei diese Stoffe in Konzentrationsbereichen von 0,5 - 40 % vorliegen.

3. Verwendung nach Anspruch 1 oder 2, dadurch gekennzeichnet, dass in dem eingesetzten topischen Trägersystem zwei Lokalanästhetika mit unterschiedlicher Pharmakokinetik kombiniert sind, wobei diese Einzelstoffe in solchen Konzentrationen vorliegen, dass die Gesamtkonzentration beider Wirkstoffe nicht mehr als 40 % beträgt.

4. Verwendung nach einem der vorhergehenden Ansprüche, dadurch gekennzeichnet, dass das topische Trägersystem in einer solchen Form vorliegt, die den speziellen Gegebenheiten des Applikationsfeldes Rückenhaut oder äußere Gelenkshaut entspricht, wobei die äußere Form des topischen Trägersystems rund, oval, eckig oder halbmondförmig, mit konkaven oder konvexen Außenformen ist, oder das Trägersystem, mit oder ohne zusätzliche Hilfsmittel, vom Anwender in entsprechende Formen segmentiert werden kann.

## Claims

1. Use of a local anesthetic for production of a composition for topical treatment of back pain, muscle tensions and myo-fascial pain, and the composition which contains local anesthetic is deposited in a topical carrier system which is suitable for intact back skin or for the outer joint skin, so that the carrier system is filled with a therapeutical dosage of the local anesthetic and capable of applying the local anesthetic targeted onto the skin region lying below the topical carrier system.

2. Use according to Claim 1, **characterised in that** in the topical carrier system are applied local anesthetics of the amide or ester type, in particular Lidocain, Tetracain, Prilocain, Bupivacain, Mepivacain, Etidocain as well as Procain and Benzocain, and these substances are present in concentrations ranging between 0.5 and 40%.

3. Use according to Claim 1 or 2, **characterised in that** in the applied topical support system are combined two local anesthetics of different pharmaco kinetics, and these individual substances are present in such concentrations that the total concentration of both active components is not more than 40%.

4. Use according to one of the above claims, **characterised in that** the topical support system is present in a form which corresponds with the special conditions of the application field of back skin or outer joint skin, and the outer form of the topical support system is round, oval, square or halfmoon-shaped, with concave or convex outside shapes, or the support system can be segmented with or without additional aids by the user into corresponding shapes.

## Revendications

1. Utilisation d'un anesthésique local pour produire un composé destiné au traitement topique des maux de dos, des crampes musculaires et des douleurs myofaciales, le composé contenant l'anesthésique local étant introduit dans un système de support topique convenant à la peau du dos intacte ou à la peau extérieure d'une articulation, de sorte que le système de support est chargé d'une dose thérapeutique de l'anesthésique local et est en mesure d'appliquer ce dernier de façon ciblée sur la région de la peau située sous le système de support topique.

2. Utilisation selon la revendication 1, caractérisée en ce que dans le système de support topique, sont introduits des anesthésiques locaux de type amide ou ester, notamment de la lidocaïne, de la tétracaïne, de la prilocaïne, de la bupyvacaïne, de la mépyvacaïne, de l'éthydocaïne ainsi que de la procaïne et de la benzocaïne, ces substances étant présentes dans des plages de concentration allant de 0,5 à 40 %.

3. Utilisation selon la revendication 1 ou 2, caractérisée en ce que dans le système de support topique utilisé, sont combinés deux anesthésiques locaux présentant une pharmacocinétique différente, ces substances individuelles se trouvant dans des concentrations telles que la concentration totale des deux substances actives ne soit pas supérieure à 40 %.

4. Utilisation selon l'une des revendications précédentes, caractérisée en ce que le système de support topique se trouve sous une forme telle qu'elle correspond aux données particulières du champ d'application, peau du dos ou peau extérieure de l'articulation, la forme extérieure du système de support topique étant ronde, ovale, rectangulaire ou en forme de demi-lune, avec des formes extérieures concaves ou convexes, ou le système de support pouvant être segmenté, avec ou sans moyen auxiliaire supplémentaire, par l'utilisateur, dans des formes convenables.
